# EUROPEAN PATENT APPLICATION

(11) **EP 3 906 799 A1**
(43) Date of publication of application: **10.11.2021**
(21) Application number: 20173066.0
(22) Date of filing: 05.05.2020
(51) Int. Cl.: A42B 3/22, A42B 3/28, A61M 16/00, A61M 16/06

(54) **FACE GUARD APPARATUS**

(71) Applicant: National University of Ireland Galway, Galway (IE)
(72) Inventor: CUMMINS, Sean, Galway (IE); O'CALLAGHAN, Rory, Galway (IE); EATON-EVANS, Jimmy, Galway (IE); LAFFEY, John, Galway (IE); JUDGE, Conor, Galway (IE)
(74) Representative: Barker Brettell LLP

(57) **Abstract**

A face guard apparatus (100) for use during the delivery of High Flow Nasal Oxygen Therapy. The face guard apparatus (100) comprising: a guard member (102) adapted to extend over at least the nose and mouth of a user, a securing device (104) adapted to secure the guard member (102) to the user, wherein at least part of a periphery (102a) of the guard member (102) is spaced apart from the head (101) of the user when the guard member (102) is secured to form an opening therebetween; and an air extraction device (106) having an air extraction opening (107) in fluid communication with a vacuum source (110) whereby, in use, air between the guard member (102) and the head of the user is drawn through the air extraction opening.

## Description

### Field of the application

The present application relates to a face guard apparatus. More specifically, the present application relates to a face guard apparatus that is suitable for use during the delivery of High Flow Nasal Oxygen Therapy to a patient. The face guard apparatus of the present application may be suitable for use during treatment of patients suffering from infectious respiratory tract diseases, including those caused by coronaviruses such as COVID-19. The face guard apparatus is however not limited to this use, and may be used in connection with the treatment of other infectious diseases and with other types of therapy.

### Background

High Flow Nasal Oxygen Therapy (HFNOT) has become an important treatment modality for patients with Acute Respiratory Failure (ARF) over the last ten years. HFNOT involves the delivery of gas flow directly to the patient via a cannula with nasal prongs that are inserted in the patient's nose. Known HFNOT devices consist of a nasal cannula, with standard sized or wide-bore prongs, connected to an oxygen flow meter with an air-oxygen gas blender and gas analyser. They offer maximum gas flow rates of between 40 and 60 litres/min, depending on the specific device. The delivered gas is heated and humidified (e.g. in a temperature range of 33-43°C and 95-100% humidity) to ensure it is well tolerated by the patient. Benefits of this therapy include reducing heat and moisture loss from the airway, reducing anatomical dead space, providing Positive End Expiratory Pressure (PEEP) as well as improved oxygenation. Patient compliance benefits also exist with this treatment modality due to the relative comfort of the nasal cannula and ability to communicate in comparison to other facemasks.

The high flows of gas associated with HFNOT present the risk of aersolisation and/or the transmission of droplets containing fluids originating from the patient. This represents an infection risk for healthcare workers operating in the vicinity of the patient, particularly in the case of treatment being provided for infectious respiratory diseases. For example, corona viruses such as COVID-19 are spread via droplets normally generated during coughing and sneezing by symptomatic patients, and which could also be generated during the delivery of HFNOT. HFNOT is known to stimulate coughing and the generation of aerosols and so its use presents a significant degree of risk to healthcare workers. Currently, some regulatory bodies recommend that HFNOT should only be delivered with airborne precautions (for example positive pressure rooms) if there is high risk of infection. The use of personal protection equipment (PPE) by healthcare workers also cannot be relied on to avoid the risk of infection.

A general problem to be addressed is therefore how to reduce the risk of infection of healthcare workers (and others) during the treatment of infectious respiratory diseases using treatments such as HFNOT.

### Summary

The present application provides a face guard apparatus for use during the delivery of High Flow Nasal Oxygen Therapy, comprising any one or more of:
a) a guard member adapted to extend over at least the nose and mouth of a user;
b) a securing device adapted to secure the guard member to the user, wherein at least part of a periphery of the guard member is spaced apart from the head of the user when the guard member is secured to form an opening therebetween; and
c) an air extraction device having an air extraction opening in fluid communication with a vacuum source, whereby, in use, air between the guard member and the head of the user is drawn through the air extraction opening.

The guard apparatus of the present application combines a guard member extending over the nose and mouth region of the user with an air extraction device arranged to draw air out of the space between the guard member and the user's face. Air is drawn from the region in front of the patient's mouth and nose into the air extraction opening thereby removing any bacterial or viral material that may be exhaled by the user. When in use, an opening is formed between the guard member and the face of the user to allow air to be entrained from the surroundings to create a flow of air able to remove infectious material.

By drawing infectious material away from the mouth and nose of the patient the risk of aerosols coming from the patient infecting healthcare workers is reduced. As discussed above, this is particularly advantageous during HFNOT treatment of infectious respiratory diseases that can be transmitted to healthcare workers through the increased risk of the patient coughing and sneezing.

The guard apparatus of the present application provides improved comfort and therefore compliance of the patient. The guard member is not sealed to the patient's face, which provides improved comfort and compliance. The lack of a seal to the face also reduces difficulty the patient may find when try to speak. The entrained air flowing from around the periphery of the guard member is advantageous in cooling the patient's face, and helps to mitigate some of the heating effects associated with HFNOT. These are otherwise uncomfortable for patients.

The air extraction device may further comprise a filter arranged to filter air drawn though the air extraction opening. This may allow aerosols from the patient to be safely trapped within the guard apparatus to reduce the risk of infection.

The filter may be a detachable filter unit. This allows the filter unit to be decoupled from the other components of the apparatus and safely disposed of.

The air extraction device may comprise a flexible hose. The air extraction opening may be fluidly connected to the vacuum source via the flexible hose. The hose may be detachably coupled (directly or indirectly) to the guard member and/or the vacuum source. This may allow the apparatus to be disassembled for storage or disposal. The filter unit may be connected at either end of the hose.

The air extraction device may comprise an elbow connector forming an angled airflow path (e.g. right angled change in direction) downstream of the air extraction opening. The elbow connector may have a connection port for the flexible hose. The hose may detachably couple to the connection port. The angled flow path reduces the risk of infectious material being ejected away from the patient if the hose were to be disconnected during use.

The connection port of the elbow connector may be directed in a downwards direction when the guard member is secured to the user. This means that any material ejected if the hose becomes disconnected is directed in a generally downwards direction away from any healthcare workers in the vicinity.

The vacuum source may be arranged to generate an air flow in a range of between 40 litres per minute and 60 litres per minute. The vacuum source may be provided by a vacuum port that is provided at a hospital bedside to provide fluid suction. This allows the guard apparatus to be easily used without a dedicated vacuum pump. Alternatively, a separate vacuum pump unit can be provided as part of the guard apparatus, or another suitable vacuum source used. In some embodiments, the vacuum source may comprise a fan (e.g. that has a propeller or impeller). The fan may be mounted on the guard member to move exhaled air through the air extraction opening (and optionally through a filter). In yet other embodiments, the vacuum source may be provided by an apparatus utilizing the Venturi effect to move air through the extraction opening and though the filter where provided.

The air extraction opening may extend through the guard member at a location opposite to the user's mount when the guard member is secured to the head of the user. This allows air from around the user's mouth and nose to be extracted.

The guard member may comprise a cover portion that acts as a barrier or shield structure extending over the face of the user. The cover portion may have a curved shape to correspond with shape of the head and face. An interior surface of the cover portion of the guard member may be approximately constant in distance from the face or head of the user. The cover portion of the guard member may extend over all of the face of the user, and partly around the sides of the head and under the chin. This provides improved capture of any aerosol material.

The guard member may comprise a bulged region in which the respective spacing between an interior wall of the guard member and the face of the user is increased. The air extraction opening may be located at a position on the bulged region. This may provide greater clearance where the extraction opening is provided to aid comfort and improve airflow.

The face guard apparatus may further comprise a mesh or grill extending over the air extraction opening. This reduces the risk of the user's lips or tongue being drawn into the opening. The mesh or grill may have a convex shape. This may prevent the user's cheek, lips etc. forming a seal over the air extraction opening.

The guard member may be formed at least partly from a transparent material. The cover member may be entirely formed from a transparent material. The use of a transparent material may aid user comfort and helps reduce the user's feeling of claustrophobia.

The guard member may comprise: a mounting portion adapted to contact the head of the user when the face guard is mounted; the cover portion shaped to extend at least partly over the face of the user and which is spaced therefore; and a step portion connecting between the mounting portion and the cover portion. The step portion may be angled related to the mounting portion so that it is shaped to provide the spacing between the cover portion and the face of the user. The separate portions of the guard member may be moulded from a single piece of material. This may aid manufacture.

The securing device may comprise a head strap arranged to extend around the head of the user to secure the guard member. The head strap may secure the guard member to the forehead of the user.

The head strap may comprise a flexible elastic strap. The head strap may be soft and flexible to aid user comfort.

The head strap may be detachable. The head strap may be coupled via a fastening such as a hook and loop fastening.

### Brief Description of the Drawings

Embodiments of the invention will now be described, by way of example only, with reference to the accompanying drawings, in which:
**Figure 1** shows a schematic view of a face guard apparatus secured to the head of a patient;
**Figure 2** shows the face guard apparatus of Figure 1 illustrating how emissions from the patient's mouth or nose are drawn into and through the extraction opening of the air extraction device; and
**Figure 3** shows a cross section through a guard member of the face guard apparatus of Figure 1.

### Detailed Description

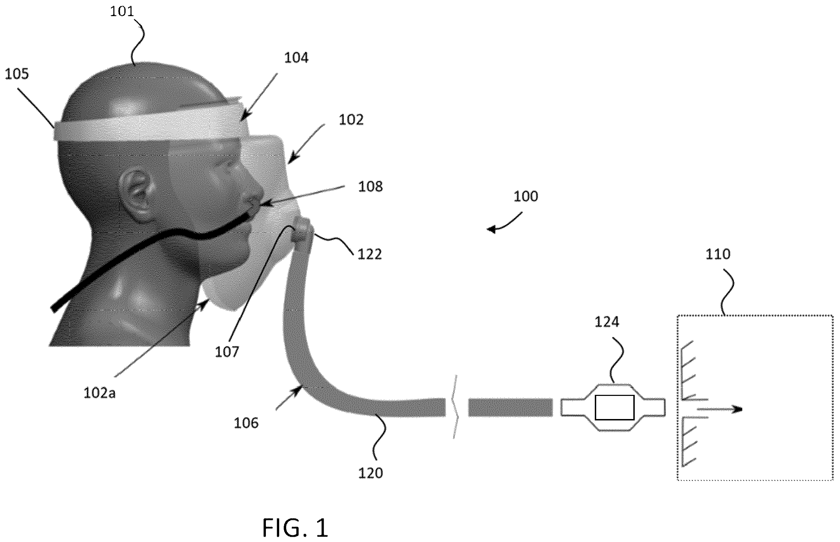

A face guard apparatus 100 is illustrated in Figure 1 secured to the head 101 of a user (i.e. a patient). The face guard apparatus 100 generally comprises a guard member 102, a securing device 104 and an air extraction device 106. The face guard apparatus 100 is specifically suited for use when a patient is receiving High Flow Nasal Oxygen Therapy (HFNOT) for infectious respiratory diseases that can be spread by aerosol emission from the patient's mouth or nose. HFNOT therapy is implemented by delivering a flow of oxygen to the patient via a nasal cannula 108 as described elsewhere herein and as known in the art. Although the face guard apparatus 100 is advantageous for use with this type of treatment, it can be used in combination with other types of therapy, or treatment of other types of disease, that is also easily spread by aerosol emission from the patent.

Referring to Figure 1, the guard member 102 is secured to the head 101 of the patient and is arranged to extend over the patient's head and face such that it is adapted to extend over at least the nose and mouth region. The guard member 102 forms a barrier to aerosols being ejected into the surrounding air if the patient were to cough or sneeze. In the embodiment shown in Figure 1, the guard member covers all of the face. In other embodiments, only part of the face may be covered so long as a barrier is formed to cover the mouth and nose region.

The guard member 102 is secured to the head 101 of the patient by the securing device 104. In the present embodiment, the securing device 104 takes the form of a head strap 105 adapted to extend around the head of the patient such that the guard member can be secured to the patient's forehead. The head strap is made from a flexible elastic material. When secured to the patient, an opening or space is provided between at least part of the periphery 102a of the guard member 102 and the user's face or head. As can be seen in Figure 1, part of the perimeter or periphery 102a of the guard member 102 is spaced apart from the user's face or head when the guard member 102 is secured to form a gap through which surrounding air may flow. In other words, the guard member 102 is not completely sealed to the user's head, but rather forms a shield extending in front of the face and defines an unsealed space between its inner surface and the patient's face and head.

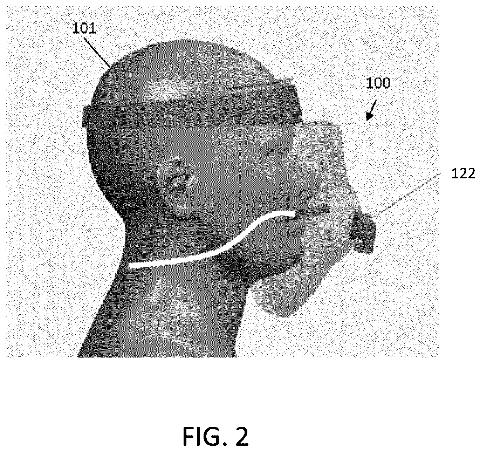

The guard apparatus 100 further comprises an air extraction device 106. The air extraction device is provided to extract air from the space between the guard member 102 and the patient's face. The air extraction device has an air extraction opening 107 that is in fluid communication with a vacuum source 110 (the vacuum source may be described as a negative pressure source). By connecting to the vacuum source the air between the guard member 102 and the face of the user is drawn through the extraction opening 107. The gap between the periphery of the guard member 102a and the patient's face allows air to be entrained from around the guard member 102 and form an air flow that is drawn through the air extraction opening 107 and is safely extracted. The scavenging of emissions from the patient's mouth in this way is illustrated in Figure 2 by an arrow showing the flow of air.

The guard apparatus 100 provides a safety system that can reduce the spread of aerosols from the patient to the surrounding air to reduce the risk of infection of healthcare workers treating the patient. The extracted air can effectively scavenge air borne droplets that otherwise cause an infection risk. The use of a guard member that does not seal to the face of the user provides improved comfort and compliance compared to a facemask that is sealed over the patent's mouth region or face. The flow of air around the guard member can further provide a cooling airflow that is advantageous during HFNOT, and help to reduce the impact on the patient's ability to speak.

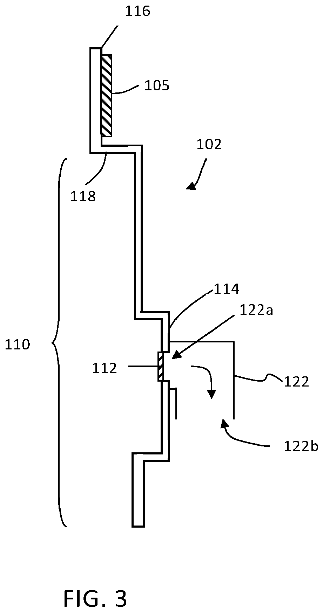

Further detail of the guard member 102 is shown in Figure 3. Referring to both Figures 1 and 3, the guard member 102 is formed from a thin sheet material that is moulded into a curved shape suitable to cover the face of the patient. In the present embodiment, the guard member 102 is attached to the forehead of the patient and comprises a cover portion 110 that extends downwards over the patient's face, including wrapping partially around the side of the head and under the chin. In other embodiments, any other suitable shape of guard member 102 may be provided so long as a suitable barrier is formed over the mouth and nose region. The cover portion 110 is mounted so that its perimeter or edge is spaced apart from the surface of the patient's face or head to provide an air gap therebetween as described above. The cover portion 110 may be formed entirely of a transparent material (e.g. a transparent polymer material) such that the patient may see easily through it. This may reduce the level of claustrophobia experienced. In other embodiments, any suitable part of the guard member 102 may be formed from the transparent material.

The air extraction opening 107 of the air extraction device 106 is formed by a through hole which extends through the guard member 102 as can be seen in Figure 3. The air extraction opening 107 is provided at a location on the guard member 102 that is directly opposite to the patients' mouth when the guard member 102 is secured to the head. This may aid collection of emissions from the patent's mouth by the airflow into the air extraction opening 107. A mesh or grill 112 extending over the air extraction opening 107 may also be provided. This may help to reduce the risk of the patient's tongue or lips being sucked into the air extraction opening. The grill may have a convex shape to prevent tissue from the patient's tongue, lips or cheeks etc. creating a seal over the extraction opening. This is advantageous as sealing of the extraction opening would bring all the negative pressure in the hospital vacuum system to bare on the patient's tissue, therefore causing trauma to said tissue. In other embodiments, the guard apparatus may comprise baffles and/or 3D shapes which may also prevent sealing over the air extraction opening.

As can be seen in Figure 1, the guard member 102 is generally shaped so that an approximately constant spacing between the interior surface of the cover portion 110 and the patient's face/head is provided. In the described embodiment, the guard member 102 comprises a bulged region 114 in which the respective spacing between an interior wall of the cover portion of the guard member 102 and the face of the user is increased compared to the surrounding parts of the guard member 102. The bulged region 114 may be positioned to align with the patient's mouth and nose region. The air extraction opening 107 is located at a position on the bulged region 114 opposite the patient's mouth.

As can be seen more clearly in Figure 3, the guard member 102 further comprises a mounting portion 116 adapted to contact the head of the user when the face guard is secured. The guard member 102 further comprises a step portion 118 between the mounting portion 116 and the cover portion 110. The step portion 118 is angled relative to the mounting portion 116 and extends in a direction away from a contact area on the patient's head or face. The step portion 118 is therefore shaped to provide the spacing between the cover portion 110 of the face guard 102 and head/face of the user. The mounting portion 116 may be pressed against the head (e.g. to the forehead) of the user by the securing device 104. The head strap 105 forming the securing device 104 extends around the head of the user and over the mounting portion 116 of the guard member 102 as shown in Figure 3 to hold it in place. The portions of the guard member described in this paragraph are formed from a single moulded piece of material to aid manufacture.

In other embodiments, an alternative means for securing the guard member 102 to the head 101 of the patient may be provided. In some embodiments, the step portion 118 may not be provided. In that case, the mounting portion 116 of the guard member 102 may comprise a spacer element that contacts the forehead of the patient and provides the spacing between the cover portion 110 and the patient's head. Alternative securing devices 104 may also be provided. In some embodiments, the head strap 105 forming the securing device 104 may be detachably coupled to the guard member 102. It may, for example, be detachably coupled using a hook and loop connector. Other suitable detachable couplings and other arrangements of strap may be provided.

Referring again to Figure 1, the air extraction device 106 will be described in more detail. As can be seen in Figure 1, the air extraction device 106 comprises a flexible hose 120 via which the air extraction opening 107 is fluidly connected to the vacuum source 110. The hose is formed by a length of tubing (e.g. 22 mm tubing). Only part of the length is shown in Figure 1. The air extraction device 106 further comprises a connector 122 having an inlet 122a and an outlet port 122b between which air flows. The connector 112 is mounted to the guard member 102 such that the inlet 122a of the connector is fluidly coupled to, or forms, the air extraction opening 107. In other embodiments, the connector 122 may extend through the thickness of the guard member 102, with the inlet 122a itself forming the air extraction opening 107 on the inside of the guard member 102. It may otherwise be mounted to the guard member in any other arrangement so that air can be extracted from the space between the guard member 102 and the patient's face. In yet other embodiments, the connector 122 may not be provided. In such an embodiment, the hose 120 (or filter unit as described later) may attach directly to a through hole in the guard member 102.

The connector 122 is formed from an elbow connector that forms an angled airflow path downstream of the air extraction opening 107. The connector 122 is thus shaped such that the inlet 122a and outlet port 122b are angled with respect to one another. In the described embodiment, the inlet 122a and outlet ports 122b are orthogonal to form a right-angled elbow connector and a right-angle change in direction of the airflow path. The air flowing out of the outlet port 122b is therefore directed in a direction along the outer surface of the guard member 102 (as shown by the arrow in Figure 3) rather than being projected perpendicularly from its surface in a direction away from the patient. This helps to reduce the risk of aerosols being projected towards a healthcare worker if the hose 120 where to become disconnected during use, especially if the patient were to cough or sneeze.

The outlet connection port 122b of the connector 122a is further orientated such that it is directed in a downwards direction when the guard member 102 is secured to the patient. This helps to direct airflow from the outlet port 122b in a downwards direction if the hose 120 were to become disconnected during use to further improve safety.

The hose 120 is detachably connected to the connector 122 so that the guard apparatus 100 can be disassembled for storage before use and for the disposal of components after use. Any suitable detachable coupling may be provided between the hose 120 and the connector 122 (or to other parts of the apparatus 100 or the vacuum source) such as a push fit or screw coupling.

The hose 120 allows the guard apparatus 100 to be coupled to a suitable vacuum source 110. This may be a vacuum source that is typically provided at a hospital bedside for use in fluid suction equipment. This allows the face guard apparatus 100 to be used with equipment usually available when HFNOT is being administered. In other embodiments, the guard apparatus 100 may comprise a separate vacuum pump unit to which the hose 120 (or filter) can be connected. In the described embodiment, the vacuum or negative pressure source is arranged to generate an air flow through the air extraction opening at a range of between 40 litres per minute and 60 litres per minute. This has been found to provide a suitable degree of airflow to remove emissions from the patient. Other types of vacuum source may be used in other embodiments.

The air extraction device 106 further comprises a filter 124 located downstream of the air extraction opening 107. The filter 124 is arranged to filter air drawn though the air extraction opening 107, thus allowing infected material to be filtered from the extracted air. This ensures that any emissions including bacterial or viral content are trapped within the filter and can be managed appropriately to safeguard healthcare workers and patients. In the presently described embodiment, the filter 124 is a separate filter unit that is detachable from the other components of the air extraction device 104. This brings particular benefits when removing the guard apparatus 100 from a patient after use and in handling of the apparatus for disassembly and disposal. The filter unit may be coupled between the vacuum source 110 and the flexible hose 120 as is illustrated in Figure 1. In other embodiments, the filter unit may be connected upstream of the hose 120, for example coupled to the outlet port 122b of the connector 122 with the hose 120 then coupled to an outlet of the filter unit 124. In yet other embodiments, the hose 122 may be divided into two lengths with the filter unit coupled between them or at any other suitable point in the airflow path.

In use, the guard member 102 of the guard apparatus 100 is secured to the head 101 of the patient as illustrated in Figure 1. The air extraction opening 107 is fluidly coupled to a vacuum source 110 by connecting the hose 120 between a suitable vacuum port provided at the patient's bedside (or dedicated vacuum pump) and the connector 122 on the guard member 102. The filter 124 is also connected in the airflow either at the connector 122 or the vacuum source as required. A continuous flow of air can then be drawn through the extraction opening to scavenge any aerosol emissions from the patient's mouth or nose, with fresh airflow also being entrained through the gap around the periphery of the cover portion of the guard member. Once treatment is finished the guard apparatus can be removed and disassembled for disposal. The guard member can also be left in place on the patient without the air extraction, or when HFNOT treatment is finished, to provide a useful barrier over the face of the patient.

Various modifications will be apparent to the skilled person without departing form the scope of the claims. The embodiments described above should be understood as exemplary only. Any feature of any of the aspects or embodiments of the disclosure may be employed separately or in combination with any other feature of the same or different aspect or embodiment of the disclosure and the disclosure includes any feature or combination of features disclosed herein.

## Claims

1. A face guard apparatus for use during the delivery of High Flow Nasal Oxygen Therapy, comprising:
a guard member adapted to extend over at least the nose and mouth of a user;
a securing device adapted to secure the guard member to the user, wherein at least part of a periphery of the guard member is spaced apart from the head of the user when the guard member is secured to form an opening therebetween; and
an air extraction device having an air extraction opening in fluid communication with a vacuum source, whereby, in use, air between the guard member and the head of the user is drawn through the air extraction opening.

2. A face guard apparatus according to claim 1, wherein the air extraction device further comprises a filter arranged to filter air drawn though the air extraction opening.

3. A face guard apparatus according to claim 2, wherein the filter is a detachable filter unit.

4. A face guard apparatus according to any preceding claim, wherein the air extraction device comprises a flexible hose, the air extraction opening being fluidly connected to the vacuum source via the flexible hose, and optionally wherein the hose is detachably coupled to the guard member and/or the vacuum source.

5. A face guard apparatus according to claim 4, wherein the air extraction device comprises an elbow connector forming an angled airflow path downstream of the air extraction opening, the elbow connector having a connection port for the flexible hose.

6. A face guard apparatus according to claim 5, wherein the connection port of the elbow connector is directed in a downwards direction when the guard member is secured to the user.

7. A face guard apparatus according to any preceding claim, wherein the vacuum source is arranged to generate an air flow in a range of between 40 litres per minute and 60 litres per minute.

8. A face guard apparatus according to any preceding claim, wherein the air extraction opening extends through the guard member at a location opposite to the user's mouth when the guard member is secured to the head of the user.

9. A face guard apparatus according to claim 8, wherein:
the guard member comprises a bulged region in which the respective spacing between an interior wall of the guard member and the face of the user is increased; and
the air extraction opening is located at a position on the bulged region.

10. A face guard apparatus according to any preceding claim, further comprising a mesh or grill extending over the air extraction opening.

11. A face guard apparatus according to any preceding claim, wherein the guard member is formed at least partly from a transparent material.

12. A face guard apparatus according to any preceding claim, wherein the guard member comprises;
a mounting portion adapted to contact the head of the user when the face guard is mounted;
a cover portion shaped to extend at least partly over the face of the user and which is spaced therefore; and
a step portion connecting the mounting portion and the cover portion, the step portion being angled related to the mounting portion so that it is shaped to provide the spacing between the cover portion and the face of the user.

13. A face guard apparatus according to any preceding claim, wherein the securing device comprises a head strap arranged to extend around the head of the user to secure the guard member.

14. A face guard apparatus according to claim 13, wherein the head strap comprises a flexible elastic strap.

15. A face guard apparatus according to claim 14, wherein the head strap is detachable, and is optionally coupled via a fastening such as a hook and loop fastening.
